# EUROPÄISCHE PATENTSCHRIFT

(11) **EP 4 518 807 B1**
(45) Veröffentlichungstag und Bekanntmachung des Hinweises auf die Patenterteilung: **21.10.2026**
(21) Anmeldenummer: 23723835.7
(22) Anmeldetag: 26.04.2023
(51) Int. Cl.: A61F 2/60, A61F 2/50

(54) **PROTHESENABDECKUNG UND VERFAHREN ZUM HERSTELLEN EINER PROTHESENABDECKUNG**
PROSTHESIS COVER AND METHOD FOR PRODUCING A PROSTHESIS COVER
ÉLÉMENT DE COUVERTURE DE PROTHÈSE ET PROCÉDÉ DE PRODUCTION D'UN ÉLÉMENT DE COUVERTURE DE PROTHÈSE

(30) Priorität: 06.05.2022 DE 102022111268
(43) Veröffentlichungstag der Anmeldung: 12.03.2025
(73) Patentinhaber: Otto Bock Healthcare Products GmbH, 1110 Wien (AT)
(72) Erfinder: LUNZER, Walter, 1110 Wien (AT); WAGNER, Sonja, 1110 Wien (AT); WILLCOCK, Angelika, 1110 Wien (AT); HOGH, Andreas, 1110 Wien (AT); BISCHOF, Johannes, 1110 Wien (AT)
(74) Vertreter: Gramm, Lins & Partner Patent- und Rechtsanwälte PartGmbB
(86) Internationale Anmeldenummer: PCT/EP2023/060999
(87) Internationale Veröffentlichungsnummer: WO 2023/213651

(56) Entgegenhaltungen:
- DE-B4- 102018 127 117
- DE-U1- 202017 000 442
- US-A1- 2007 162 154

## Beschreibung

Die Erfindung betrifft eine Prothesenabdeckung mit zumindest einer Befestigungseinrichtung zum Festlegen der Prothesenabdeckung an einer Prothesenkomponente und einem flexiblen Grundkörper, mit einer der Prothesenkomponente im angelegten Zustand zugewandten Innenseite und einer der Innenseite gegenüberliegenden Außenseite, an dem die zumindest eine Befestigungseinrichtung angeordnet ist. Die Erfindung betrifft ebenfalls ein Verfahren zum Herstellen einer Prothesenabdeckung mit zumindest einer Befestigungseinrichtung zum Festlegen der Prothesenabdeckung an einer Prothesenkomponente und einem flexiblen Grundkörper, mit einer der Prothesenkomponente im angelegten Zustand zugewandten Innenseite und einer der Innenseite gegenüberliegenden Außenseite, an dem die zumindest eine Befestigungseinrichtung angeordnet ist.

Prothesenabdeckungen oder Prothesenkosmetiken dienen zur Verkleidung einer Prothesenkomponente unter anderem mit dem Ziel, eine möglichst natürliche Anmutung der ersetzten Gliedmaße zu erreichen und um die Prothesenkomponente vor äußeren Einflüssen zu schützen. Darüber hinaus schützen Prothesenkosmetiken und Prothesenabdeckungen die Umgebung vor einem unmittelbaren Kontakt mit der Prothesenkomponente und haben darüber hinaus eine ästhetische Komponente.

Prothesenabdeckungen können aus einem Schaumwerkstoff hergestellt werden, wobei die Anfertigung solcher Prothesenabdeckungen aufwendig ist. Bei der Herstellung wird ein Grundkörper aus einem Schaumwerkstoff mit einem Hohlraum zur Aufnahme der Prothesenkomponente durch Abtragen von Material an den jeweiligen Patienten angepasst. Das Abtragen erfolgt überwiegend durch Schleifen oder durch andere Trennverfahren und benötigt eine große Erfahrung und vergleichsweise viel Zeit von einem Orthopädietechniker. Aufgrund der bei dem Schleifen entstehenden rauen Oberfläche können solcher Prothesenkosmetiken noch nicht in einer annehmbaren Art und Weise eingesetzt werden, vielmehr ist es notwendig, über die Außenoberfläche einen weiteren Überzug zu ziehen, beispielsweise aus einem elastischen Stoffmaterial.

Neben der Ausgestaltung von Prothesenabdeckungen aus einem Schaumaterial existieren schalenartige Prothesenabdeckungen, die die Funktionselemente einer Prothese abdecken und eine glatte Außenseite aufweisen. Diese Prothesenabdeckungen bestehen aus Kunststoff, einem Verbundwerkstoff oder einem Metall und haben eine eher technische Anmutung.

Aus der DE 10 2018 127 117 A1 ist eine Prothesenverkleidung sowie ein Verfahren zu deren Herstellung bekannt, bei dem ein formstabiler Grundkörper mit einem Hohlraum zur Aufnahme einer Prothesenkomponente in einem additiven Herstellerverfahren erzeugt wird. Der Grundkörper ist einteilig ausgebildet und weist einen offenen Querschnitt mit in Längserstreckung der Prothesenverkleidung orientierten Stoßkanten auf. Die Prothesenverkleidung ist entlang ihrer Längserstreckung aufbiegbar ausgebildet. Der Grundkörper kann eine Gitterstruktur aufweisen und im Wesentlichen der Kontur der durch die Prothesenkomponente ersetzten Gliedmaße entsprechen.

Aus der US 2007/0 162 154 A1 ist eine Prothesenabdeckung mit einem hohlen, flexiblen Grundkörper bekannt, die eine im Wesentlichen zylindrische Grundform aufweist. Eine Naht erstreckt sich von einer unteren Öffnung zu der oberen Öffnung, um die Abdeckung um die Prothesenkomponente herum anlegen zu können. Als Verschlusseinrichtung ist beispielsweise ein Klettverschluss vorgesehen. Das Material des Grundkörpers ist Neopren.

Die DE 10 2018 132 640 A1 betrifft ein elastisches Flächenmaterial und ein Verfahren zu dessen Herstellung bei dem ein Funktionselement mit dem Flächenmaterial verbunden wird und zumindest ein Begrenzungselement zwischen dem Funktionselement und dem Flächenmaterial oder auf der dem Funktionselement gegenüberliegenden Seite des Flächenmaterials angeordnet ist. Das Begrenzungselement verringert die Elastizität des Flächenmaterials und erhöht die Haftung des Funktionselementes aufgrund der verringerten Relativbewegung bei der Benutzung des Flächenmaterials. Das Funktionselement kann in einem additiven Herstellerverfahren auf das Flächenmaterial oder das Begrenzungselement aufgebracht werden.

Die WO 2022/024114 A1 betrifft ein System und ein Verfahren zum 3D-Bedrucken von Textilien, bei dem ein Areal von Düsen zum Aufbringen des zu druckenden Materials oberhalb eines Bedruckstoffes angeordnet wird. Über eine Computersteuerung werden die Düsen aktiviert, um die gewünschte Form eines Objektes auf dem Textil herzustellen. Zum Aushärten des Materials wird dieses erwärmt oder mit UV-Licht bestrahlt.

Die DE 20 2017 000 442 U1 betrifft ein zweiteiliges Kugelgelenk für die Prothetik, das von einer kosmetischen Verschalung umgeben ist. Die kosmetische Verschalung ist eine Kunststoffverschalung aus einem harten Kunststoff, beispielsweise Polyamid, und ist in einem additiven Fertigungsverfahren hergestellt. Die Kunststoffverschalung besteht aus zwei Hälften, die mittels zweier äußerer, kalottenförmiger Anbindungselemente an der Prothese festgelegt werden. Dazu sind an der Innenseite der Kunststoffverschalungen federnde Laschen angeordnet.

Die individuelle Anfertigung von Prothesenabdeckungen ist weiterhin mit einem hohen Aufwand verbunden, beispielsweise über die Aufnahme von Daten mit einem 3D-Scanner und der individuellen Anfertigung von Prothesenabdeckungen. Darüber hinaus ist es nicht möglich, bereits bestehende Prothesenabdeckungen zu modifizieren oder zu individualisieren.

Aufgabe der vorliegenden Erfindung ist es, eine Prothesenabdeckung und ein Verfahren zu dessen Herstellung bereitzustellen, die eine Individualisierung oder individualisierte Prothesenabdeckungen ermöglichen.

Diese Aufgabe wird durch eine Prothesenabdeckung mit den Merkmalen des Hauptanspruches sowie ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die Prothesenabdeckung mit zumindest einer Befestigungseinrichtung zum Festlegen der Prothesenabdeckung an einer Prothesenkomponente und einem flexiblen Grundkörper, mit einer der Prothesenkomponente im angelegten Zustand zugewandten Innenseite und einer der Innenseite gegenüberliegenden Außenseite, an dem die zumindest eine Befestigungseinrichtung angeordnet ist, sieht vor, dass auf der Außenseite zumindest eine von der Außenseite abstehendes Strukturelement in einem additiven Fertigungsverfahren aufgebracht ist. Über additiven Fertigungsverfahren, beispielsweise die verschiedenen Varianten eines 3-D-Druckes, ist es möglich, eine Vielzahl unterschiedlicher Strukturelemente in unterschiedlichen Formen, Anordnungen, Designs und in unterschiedlichen Materialien auf einem flexiblen, insbesondere elastischen Grundkörper, insbesondere einem Textil aufzubringen und über die Befestigungseinrichtung an einer Prothesenkomponente festzulegen. Die Prothesenabdeckung oder Prothesenkosmetik kann aus dem Grundkörper und der Befestigungseinrichtung bestehen. Während bei herkömmlichen Prothesenabdeckungen Spritzgussteile verwendet werden, die um die standardisierten Prothesenkomponenten herum angeordnet werden können, was einen hohen Investitionsaufwand für die Werkzeuge erfordert, ist es mit der vorgeschlagenen Prothesenabdeckung möglich, mit geringen Werkzeugkosten Prothesenabdeckungen für unterschiedlichste Prothesen und in unterschiedlichsten Formen zu individualisieren oder individuell herzustellen.

In einer Ausgestaltung ist die Prothesenabdeckung mit mehreren Strukturelementen versehen, die voneinander beanstandet an dem Grundkörper aufgebracht sind. Die Strukturelemente können in unterschiedlichen Abständen zueinander, in Gruppen, in Gruppen mit unterschiedlichen Abständen zueinander und/oder in Gruppen mit unterschiedlichen Abständen auf dem Grundkörper angeordnet sein. Die Strukturelemente können gleiche oder unterschiedliche Höhen aufweisen, also unterschiedlich hoch von der Außenseite abragen. Die einzelnen Gruppen können Strukturelemente mit gleichen oder unterschiedlichen Höhen aufweisen. Es können mehrere Gruppen mit einem gleich ausgebildeten Höhenprofil vorhanden sein. Ebenso ist es möglich, dass alle Strukturelemente gleich beanstandet zueinander auf der Außenseite aufgebracht sind und ein Höhenprofil bilden, das aus Strukturelementen mit zumindest teilweise unterschiedlichen Höhen besteht.

Die Strukturelemente können in regelmäßigen und/oder unregelmäßigen Formen ausgebildet sein, beispielsweise als Halbkugeln, Kegel, Pyramiden, Kegelstümpfe, Pyramidenstümpfe, in Tropfenform, als Platten, Würfeln, Noppen oder anderen, beliebigen geometrischen Formen.

Durch die variable Anordnung und/oder Formen der Strukturelemente können einerseits ein individuelles Erscheinungsbild und andererseits gewünschte Funktionen wie Schutz, Belüftung, Haptik etc. erreichen werden.

Die Strukturelemente sind in einer Ausgestaltung mehrfarbig und/oder aus unterschiedlichen Materialien ausgebildet. Insbesondere sind die Materialien, die an der Außenseite des Grundkörpers anhaften, farbig und undurchsichtig oder nur teiltransparent ausgebildet, während die weiter entfernt von dem Grundkörper aufgebrachten Schichten des Materials der Strukturelemente transparent oder zumindest teiltransparent sind. In einer Variante sind die Strukturelemente vollfarbig ausgebildet. Auch die Außenseite kann vollfarbig und gegebenenfalls undurchsichtig ausgestaltet sein. Durch die unterschiedlichen Materialien und/oder unterschiedlichen Farbgebungen können Individualisierungseffekte einfach erzielt werden.

Die Strukturelemente sind in einer Ausgestaltung aus einem elastischen Material ausgebildet oder weisen zumindest ein elastisches Material oder eine elastische Materialkomponente auf, insbesondere sind die Außenschichten oder ist die Außenschicht aus einem elastischen Material ausgebildet. Die Außenschicht ist insbesondere durchsichtig oder teildurchsichtig.

In einer Ausgestaltung der Prothesenabdeckung durchdringt das Material der Strukturelemente den Grundkörper nicht, sodass auf der Innenseite des Grundkörpers lediglich das Material des Grundkörpers zu spüren ist. Hierzu ist es insbesondere vorteilhaft, wenn der Grundkörper aus einem Material besteht oder ein Material aufweist, das eine hohe Oberflächenenergie aufweist. Materialien mit einer hohen Oberflächenenergie erleichtern das Anhaften des Materials der Strukturelemente, beispielsweise eines Harzes, das für die additive Fertigung verwendet wird. Bei der Verwendung von textilen Grundkörpern oder von einem Grundkörper mit einem Textilanteil werden bevorzugte Materialien mit einer hohen Oberflächenenergie eingesetzt, beispielsweise Polyamide oder Baumwolle. Insbesondere bei textilen Komponenten spielt die Rauigkeit der Rohstoffe oder die Haarigkeit des Garns eine Rolle bei der Haftfähigkeit und der Adhäsion des Materials der Strukturelemente. Je größer die reale Kontaktfläche zwischen dem Material des Grundkörpers und dem Material der Strukturelemente ist, desto größer ist die Adhäsion des Materials der Strukturelemente.

In einer Ausgestaltung ist der Grundkörper der Prothesenabdeckung elastisch und/oder aufnahmefähig oder saugfähig für das mit dem Grundkörper in Kontakt stehende Material der Strukturelemente ausgebildet, sodass das aufgetragene oder aufgedruckte Material vor dem eigentlichen Härtungsvorgang verläuft und in den Grundkörper einsinkt oder eindringt. Dabei wird bevorzugt ein Polymer im Rahmen eines 3D-Drucks aufgebracht, das mit UV-Strahlen ausgehärtet wird. In einer Ausgestaltung wird das Material über mehrere Düsen auf eine Werkplattform aufgetragen und das noch flüssige Material ausgehärtet, bevor es durch den Grundkörper durchdringen kann. Zur oder nach der Aushärtung fährt die Plattform mit dem Grundkörper um die Dicke der aufgetragenen Schicht nach unten bzw. der Druckkopf mit den Druckdüsen nach oben. Anschließend wird eine weitere Schicht des aufzutragenden Materials in der gewünschten Form und in der gewünschten Dicke auf die bereits ausgehärtete Schicht aufgetragen und dies so lange wiederholt, bis die gewünschte Form des Strukturelementes gedruckt worden ist. Das aufgetragene Material für die Strukturelemente ist vorteilhafterweise flüssig und wird durch Düsen aufgetragen, sodass es vor dem Härtungsvorgang verläuft. Dadurch ist es möglich, eine sehr glatte Oberfläche der Strukturelemente zu erreichen, ohne dass eine Nachbearbeitung notwendig wird. Insbesondere weist die Vorrichtung zum Drucken und Auftragen der Materialien mehrere Druckköpfe auf, sodass mehrere Materialien verwendet werden können und/oder in mehreren Farben gedruckt werden kann.

Der Grundkörper weist in einer Ausgestaltung mehrere Abschnitte aus unterschiedlichen oder gleichartigen Materialien, insbesondere Textilien, oder einer Kombination aus Textilien mit beispielsweise anderen Kunststoffmaterialien, Naturmaterialien, Pappe, Metallen oder Verbundwerkstoffen auf, sodass ein Materialmix entsteht oder ein Grundkörper aus einem Materialtyp in unterschiedlichen Ausgestaltungen bestehen kann. Beispielsweise können bei einem textilen Grundkörper unterschiedliche Materialien an Fäden oder Garnen, unterschiedliche Fadenstärken, Webarten, Strickarten, Gewebebindungen, Materialqualitäten oder Farben sowie Materialmixe eingesetzt werden, die dann mit den Strukturelementen versehen werden. Als Garne oder Fäden können auch Filamente und Spinnfasergarne verwendet werden, die Filamente können in unterschiedlichen Verfahren hergestellt werden, beispielsweise in einem Schmelzspinnverfahren, einem Trockenspinnverfahren und einem Nassspinnverfahren mit gegebenenfalls anschließender Texturierung.

Die verschiedenen Abschnitte des Grundkörpers können aus unterschiedlichen Materialien bestehen oder Materialabweichungen aufweisen, auf denen unterschiedliche Anordnungen und/oder Formen von Strukturelementen angebracht sind.

Der Grundkörper weist in einer Ausgestaltung einen proximalen und/oder distalen Halter oder Rahmen auf und kann unmittelbar an der Prothesenkomponente oder einem formstabilen Teil der Prothese, zum Beispiel einem Rohr oder einer Gelenkkomponente angeordnet werden. Der Grundkörper kann auch auf einem Träger, insbesondere einem schalenartigen Träger angeordnet werden, beispielsweise einem Protektor, sodass über die Befestigungseinrichtung die jeweilige äußere Oberfläche verändert und individualisiert werden kann. Die Befestigungseinrichtung kann beispielsweise eine Klebeschicht sein, die auf den insbesondere schalenartigen Träger aufgeklebt wird. Der Träger selbst kann auch eine Befestigungseinrichtung oder ein Befestigungselement aufweisen, um ihn an der Prothese oder einer Prothesenkomponente festzulegen. Der Grundkörper kann lösbar an dem Träger und gegebenenfalls an dem Halter oder Rahmen festgelegt sein, beispielsweise über Kraftschlusselemente wie Magnete oder Formschlusselemente wie Klettverschlüsse, Klemmeinrichtungen, Haken, Druckknöpfe, Reißverschlüsse oder dergleichen.

In einer Ausgestaltung ist der Halter an dem Grundkörper angenäht, angeklebt, angeschweißt, angespritzt oder durch Umspritzen an dem Rahmen dauerhaft befestigt.

Das Verfahren zum Herstellen einer Prothesenabdeckung mit zumindest einer Befestigungseinrichtung zum Festlegen der Prothesenabdeckung an einer Prothesenkomponente und einem flexiblen Grundkörper, insbesondere mit einer textilen Komponente, mit einer der Prothesenkomponente im angelegten Zustand zugewandten Innenseite und einer der Innenseite gegenüberliegenden Außenseite, an dem die zumindest eine Befestigungseinrichtung angeordnet ist, sieht vor, dass an der Außenseite oder auf der Außenseite zumindest ein von der Außenseite abstehendes Strukturelement in einem additiven Fertigungsverfahren aufgebracht wird. Der Grundkörper kann mit dem zumindest einen Strukturelement an einem Halter oder Träger befestigt werden, beispielsweise lösbar über Formschlusselemente oder Kraftschlusselemente oder alternativ über eine stoffschlüssige Befestigung, beispielsweise durch Kleben oder Schweißen.

Sofern der Grundkörper eine textile Komponente aufweist, ist zur Erhöhung der Adhäsion eine Ausgestaltung der textilen Komponente mit glatten Bindungen, wie zum Beispiel eine Atlasbindung, vorteilhaft, da solche glatten Bindungen eine höhere reale Kontaktfläche als zum Beispiel eine Körperbindung bei einem Jeansstoff aufweisen. Sofern ausreichend Hohlräume zwischen einzelnen Maschen vorhanden sind, erlauben Maschenwaren ein Durchdringen des Materials der Strukturelemente, insbesondere eines aushärtbaren Harzes mit dem Textil und eine sehr gute Anhaftung an dem Grundkörper. Zur Erhöhung der Oberflächenenergie kann der Grundkörper oder das Material des Grundkörpers beschichtet sein, beispielsweise im Rahmen einer Plasmabeschichtung oder einer Hydrophilierung über Tenside.

Der Grundkörper weist in Ausgestaltung statt oder neben einer textilen Komponente, alternative Werkstoffe wie Kork, Kunstleder, Leder, Schichtwerkstoffe, Pappe und auch Gitterwerkstoffe, Netze oder Vliesmaterial auf, um weitere Gestaltungseffekte durch den Grundkörper und das Grundkörpermaterial bereitstellen zu können. Die Prothesenabdeckung kann auch an bereits bestehende Prothesenkomponenten aufgebracht werden, beispielsweise aufgeklebt, aufgezogen, per Magnet gehalten oder mit anderen Befestigungseinrichtung oder Befestigungsmitteln an der Prothesenkomponente oder einer anderen Prothesenabdeckung angebracht werden. Die Prothesenabdeckung kann neben einer Abdeckung für Prothesenkomponenten der unteren Extremitäten auch für eine Prothesenabdeckung für Prothesen der oberen Extremitäten vorgesehen sein, beispielsweise als Überzug für einen Unterarmschaft oder als Prothesenhandschuh.

Bei einer Textilkomponente weist das verwendete Material insbesondere ein texturiertes Polyamid in Form von Maschenware auf, die am besten einbettig gestrickt oder gewirkt ist.

Die Strukturelemente sind vorteilhafterweise an denjenigen Stellen der Prothesenabdeckung angeordnet, an denen sie eine zusätzliche, insbesondere schützende Funktion haben, sodass eine Materialverstärkung an besonders beanspruchten Stellen erreicht werden kann. Durch die im additiven Fertigungsverfahren aufgedruckten Strukturelementen kann zudem eine verbesserte Isolierung, ein mechanischer Schutz und/oder eine verbesserte Kühlung sowie eine verbesserte Haptik und Optik der Prothesenabdeckung erreicht werden.

**In** einer Ausgestaltung ist das aufgedruckte Material der Strukturelemente vor dem Aushärten flüssig oder gelartig und ermöglicht es dadurch, dass das Material des Grundkörpers auch elastisch ausgebildet sein kann. Dadurch ist es möglich, das Material des Grundkörpers vor dem Bedrucken und während des Bedruckens zu dehnen und nach dem Bedrucken zu entspannen.

Die Strukturelemente sind in einer Ausgestaltung so weit voneinander beanstandet, dass die grundsätzlichen Eigenschaften des Grundkörpers, insbesondere hinsichtlich der Flexibilität und Elastizität, sich durch die Strukturelemente nicht oder nahezu nicht verändern. Ein weiterer Vorteil des Verfahrens und der Prothesenabdeckung besteht darin, dass bisherige additive Fertigungsprozesse primär zur Erstellung von Prototypen herangezogen worden ist, sodass der Aspekt der Haltbarkeit nicht berücksichtigt wurde. Mit dem vorliegenden Verfahren und dem vorliegenden Produkt ist es möglich, eine dauerhafte Haltbarkeit der Strukturelemente an dem Produkt zu erzielen und eine effiziente und ökonomische Herstellung von Prothesenabdeckungen bereitstellen zu können. Die Verbindung des aufgebrachten Materials mit dem Grundkörper ist haltbar, wird insbesondere bislang bei dem Bedrucken von Grundkörpern aus textilen Materialien die Haftung unbefriedigend war und die textilen Flächen speziell ausgerüstet werden mussten.

Nachfolgend werden Ausführungsbeispiele der Erfindung anhand der Figuren näher erläutert. Es zeigen:
Figur 1 - drei Darstellungen Prothesen mit Prothesenabdeckung;
Figur 2 - eine Variante der Prothesenabdeckung;
Figur 4 - eine Einzeldarstellung eines Grundkörpers mit Strukturelementen;

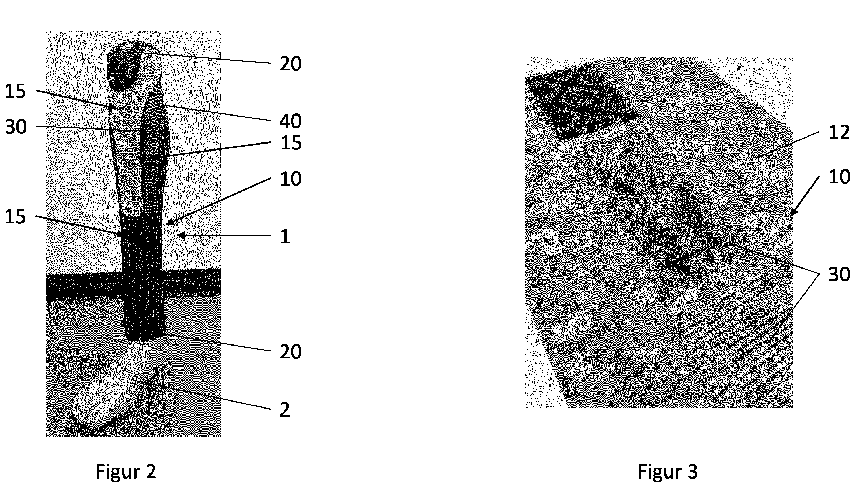

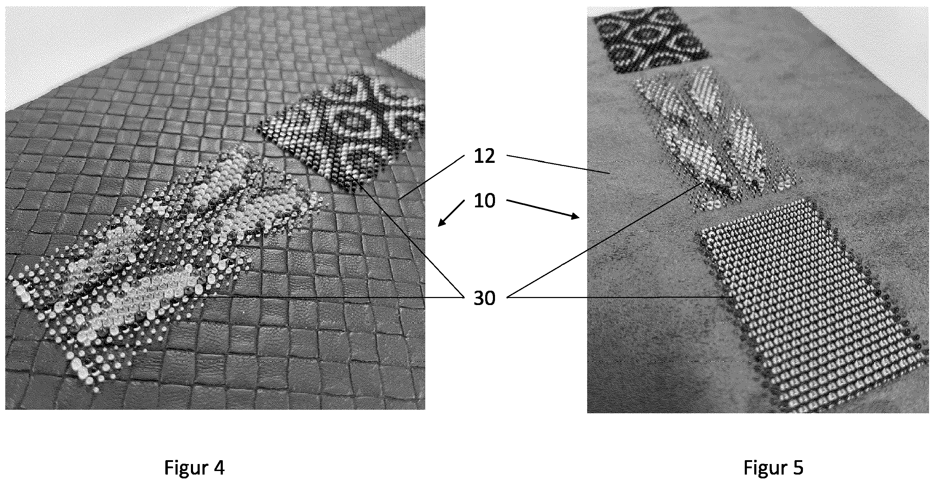

Figur 4 - eine Variante der Figur 3 mit einem Grundkörper aus Leder;
Figur 5 - eine Variante der Figur 3 mit einem Grundkörper Baustoff; sowie

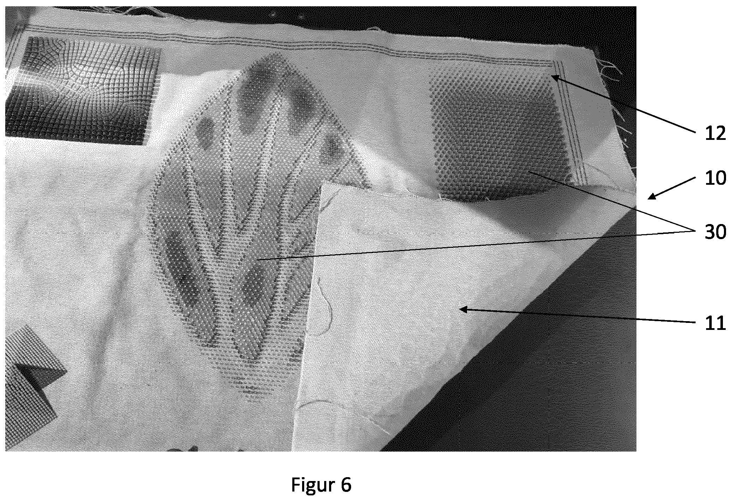

Figur 6 - eine Detailansicht eines Grundkörpers mit Strukturelementen.

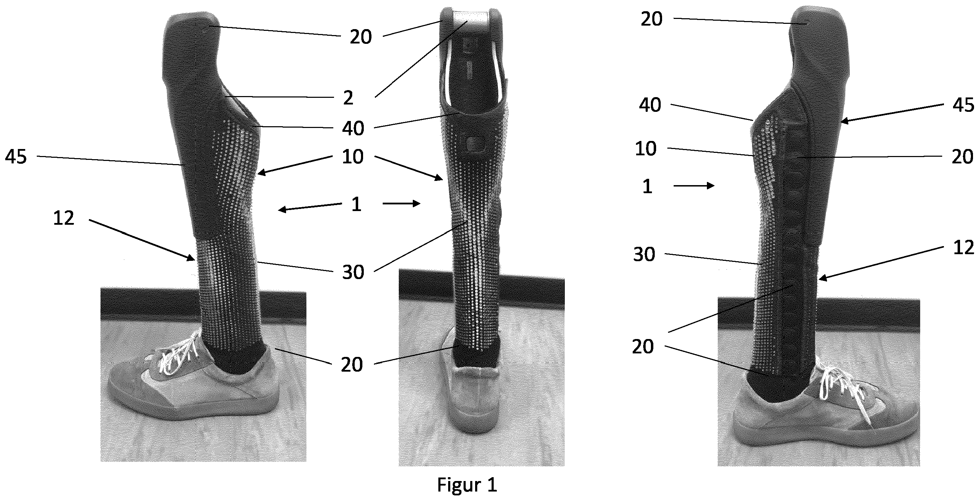

**In** der Figur 1 sind drei Ansichten einer Prothesenabdeckung 1 dargestellt, von links nach rechts in lateraler, posteriorer und medialer Ansicht. Die Prothesenabdeckung 1 ist an einer Prothesenkomponente 2, die in dem dargestellten Ausführungsbeispiel als Unterschenkelprothese ausgebildet ist, befestigt. Die Prothesenabdeckung 2 weist einen flexiblen Grundkörper 10 aus einem textilen Material auf. Der Grundkörper 10 kann auch elastisch ausgebildet sein. Die Prothesenabdeckung 10 ist über eine Befestigungseinrichtung 20 an der Prothesenkomponente 2 in Gestalt eines Prothesenkniegelenkes befestigt. Das Prothesenkniegelenk weist zwei seitliche Befestigungsteile oder Befestigungspunkte auf, mit denen die Befestigungseinrichtung 20 wechselwirkt oder in Eingriff tritt. Diese Befestigungseinrichtung 20 ist als eine Kappe ausgebildet, die an dem proximalen Ende der Prothesenabdeckung 1 angeordnet ist. Die Kappe ist schalenartig ausgebildet und erstreckt sich medial und lateral sowie frontal über einen Teil der Prothesenkomponente 2. Die Befestigungseinrichtung 20 kann aufgebogen oder auf eine andere Art und Weise geöffnet und um die Prothesenkomponente 2 herumgelegt werden. Distal an die Befestigungseinrichtung 20 schließt sich ein Träger 45 an, der schalenartig ausgebildet ist und sich in dem Bereich des Schienbeines in distaler Richtung erstreckt. Der Träger 45 reichte bis ungefähr zu der Mitte des Unterschenkels. Von dem Träger 45 verläuft ein Halter 40 oder Rahmen an dem proximalen, hinteren oder posterioren Abschluss des Grundkörpers 10. Der Halter 40 ist ein biegsames, jedoch formstabiles Bauteil, das den proximalen, posterioren Abschluss des Grundkörpers 10 und der Prothesenabdeckung 1 bildet.

Der Grundkörper 10 kann an dem Halter 40 angeschweißt, angenäht, angeklebt, angeklemmt oder auf eine andere Art und Weise dauerhaft oder lösbar daran befestigt sein. An dem Träger 40 ist der Grundkörper 10 ebenfalls befestigt. **In** dem dargestellten Ausführungsbeispiel ist der Grundkörper 10 an dem Träger 45 an der lateralen Seite dauerhaft und unlösbar befestigt, beispielsweise angeklebt, angeschweißt, angespritzt oder durch Formschlusselemente daran fixiert. Alternativ ist der Grundkörper 10 lösbar an dem Träger 45 befestigt, beispielsweise über Formschlusselemente in Gestalt von Clipsen, Klettverschlüssen, Haken, Hinterschnitten oder dergleichen.

**In** der rechten Darstellung der Figur 1 ist zu erkennen, dass an der medialen Seite des Grundkörpers 10 Befestigungseinrichtungen 20 von proximal nach distal verlaufend angeordnet sind. In dem dargestellten Ausführungsbeispiel sind die Befestigungseinrichtungen 20 als Magnete ausgebildet, die an einem seitlichen Rand des Grundkörpers 10 angeordnet sind. Über die Befestigungseinrichtungen 20 wird der Grundkörper 10 an der medialen Seite an dem Träger 45 und/oder der Protheseneinrichtung 2 reversibel lösbar befestigt. Die Befestigung erfolgt kraftschlüssig. Alternativ zu einer Ausgestaltung der Befestigungseinrichtungen 20 als Magnete können die Befestigungseinrichtungen 20 auch als Reißverschluss, Klettverschluss oder andere formschlüssige und/oder kraftschlüssige Befestigungseinrichtungen ausgebildet sein. Distal zu dem Träger 45 wird der zunächst offene Querschnitt des Grundkörpers 10 auf sich selbst gelegt und geschlossen, sodass sich eine von proximal nach distal verjüngende, im Wesentlichen zylindrische und hohle Grundform der Prothesenabdeckung 20 ergibt, innerhalb der die Prothesenkomponente 2 angeordnet ist.

An dem distalen Ende des Grundkörpers 10 sind ebenfalls Befestigungseinrichtungen 20 angeordnet oder ausgebildet, um den Grundkörper 10, beispielsweise an einem Prothesenfuß, einer Prothesenfußabdeckung oder einer anderen Komponente der Prothese festlegen zu können.

Alternativ oder in Ergänzung zu der Anordnung neben und hinter dem Träger 45 kann der Grundkörper 10 auch an der Außenseite des Trägers 45 angeordnet, insbesondere aufgeklebt sein.

Der Grundkörper 10 ist in dem dargestellten Ausführungsbeispiel aus einem flexiblen und elastischen Textil hergestellt, an dessen Außenseite 12 Strukturelemente 30 im Rahmen eines additiven Fertigungsverfahrens aufgebracht sind. Die Außenseite 12 liegt auf der der Prothesenkomponente 2 abgewandten Seite des Grundkörpers 10. Strukturelemente sind in dem dargestellten Ausführungsbeispiel der Figur 1 aus einem transparenten bzw. teiltransparenten Material ausgebildet und weisen unterschiedliche Größen auf. Sowohl die Durchmesser als auch die Höhen der einzelnen Strukturelemente 30 sind verschieden, wobei einige Strukturelemente 30 den gleichen Durchmesser und die gleiche Höhe aufweisen. Die Strukturelemente 30 sind bereichsweise mit unterschiedlichen Durchmessern und Höhen versehen, um einen individualisierten Gesamteindruck sowie eine verbesserte Funktionalität in besonders belasteten Bereichen bereitstellen zu können.

**In** der Figur 2 ist eine perspektivische Gesamtansicht einer Prothesenabdeckung 1 mit einem textilen Grundkörper 10 dargestellt. Auch hier handelt es sich um eine Prothesenabdeckung 10 für eine Unterschenkelprothese mit einem Prothesenkniegelenk und einem Prothesenfuß. Die Prothesenabdeckung 1 ist an dem proximalen Ende mit der Befestigungseinrichtung 20 in Gestalt einer Kniekappe an der Prothesenkomponente 2 im proximalen Bereich festgelegt. Von der Befestigungseinrichtung 20 erstreckt sich ebenfalls ein Halter 40 in posteriorer Richtung. Der Grundkörper 10 umgibt in dem fertig angelegten Zustand die Prothesenkomponente in dem Unterschenkelbereich umfänglich. Je nach Ausgestaltung des Materials des Grundkörpers 10 kann die Prothesenabdeckung 1 über die Prothesenkomponente gezogen oder ist mit einem offenen Querschnitt ausgebildet und kann über Befestigungseinrichtungen, wie sie in der Figur 1 erklärt worden sind, geschlossen werden. Der Grundkörper 10 in dem Ausführungsbeispiel weist mehrere Abschnitte 15 aus unterschiedlichen Materialien auf, die unterschiedliche Formen, Farben und Funktionalitäten aufweisen. Ein erster Abschnitt 15 kann mit einer anderen Farbe ausgebildet sein, als ein zweiter Abschnitt. Ein dritter Abschnitt 15 im distalen Bereich weist eine besondere Flexibilität auf und kann mit Stabilisierungseinrichtungen oder Strukturelementen 30 in Längserstreckung ausgestattet sein. Jeder Abschnitt 15 weist unterschiedliche Strukturelemente 30 an der jeweiligen Außenseite auf, wobei hinter dem Grundkörper 10 der Träger 45 im Schienbeinbereich zur Stabilisierung und Formgebung angeordnet sein kann. Die Abschnitte 15 können aus unterschiedlichen Materialien hergestellt sein. Dabei sind unterschiedliche Materialien auch gleichartige Materialien, beispielsweise Textilien, die unterschiedliche Eigenschaften aufweisen. Unterschiedlichen Eigenschaften werden durch unterschiedliche Garne, Fadenstärken, Web- oder Strickarten und/oder veränderte Oberflächeneigenschaften erzeugt. Es besteht auch die Möglichkeit, einen Mix aus nicht gleichartigen Materialien bereitzustellen, beispielsweise Textilabschnitte 15 in Verbindung mit Abschnitten aus Leder, Kunststoff und/oder Metall.

In der Figur 3 ist eine Detailansicht eines Grundkörpers 10 aus einem Korkmaterial dargestellt, auf dessen Außenseite 12 verschiedene Strukturelemente 30 über eine additives Fertigungsverfahren aufgebracht worden sind. Die Strukturelemente 30 weisen eine Tropfenform auf und sind beanstandet zueinander auf der Außenseite 12 befestigt. In dem dargestellten Ausdrucksbeispiel sind drei Areale mit Strukturelementen 30 versehen. Ein erstes Areal weist rein transparente Strukturelemente 30 auf, die gleichbeanstandet und gleichförmig zueinander ausgebildet und auf der Außenseite 12 angeordnet sind. In dem mittleren Areal sind tropfenförmige Strukturelemente 30 in unterschiedlichen Durchmessern, mit unterschiedlichen Höhen und mit unterschiedlichen Farben angeordnet. Zumindest eine Außenseite oder Außenschicht der Strukturelemente 30 ist transparent. In dem dritten Bereich sind gleichartige aber unterschiedlich gefärbte Strukturelemente 30 auf der Außenseite 12 aufgebracht.

In den Figuren 4 und 5 sind die Bereiche mit den Strukturelementen 30 auf unterschiedlichen Materialien der Grundkörper 10 aufgebracht. Das Design der Strukturelemente 30 ist dabei gleich geblieben, in der Figur 4 ist das Material des Grundkörpers 10 Leder, in der Figur 5 ist es ein Veloursstoff.

In der Figur 6 ist eine Grundkörper an 10 mit einer Außenseite 12 mit Strukturelementen 30 dargestellt, die in einem additiven Fertigungsverfahren auf die Außenseite 12 aufgebracht worden sind. Ein Teil des Grundkörpers 10 ist umgeschlagen, sodass die Innenseite 11 des Grundkörpermaterials zu erkennen ist. Auf der Innenseite 11 ist zu erkennen, dass kein Material der Strukturelemente 30 den Grundkörper 10 durchdrungen hat, so dass die Innenseite 11 des Grundkörpers 10 frei von dem Material der Strukturelemente 30 ist.

In der Figur 6 sind vier unterschiedliche Bereiche mit unterschiedlichen Formen, Größen, Farben und unterschiedlichen Abständen von Strukturelementen 30 zueinander zu erkennen. Strukturelemente 30 in gleicher Form können in unterschiedlichen Farben auf der Außenseite 12 aufgebracht worden sein. Zumindest eine Farbschicht, in der Regel mehrere Schichten des schichtweise aufgebrachten Materials der Strukturelemente 30, sind farbig und werden von transparenten oder teiltransparenten weiteren Schichten und einer Außenschicht umgeben oder abgedeckt. Strukturelemente 30 können unterschiedlich beabstandet zueinander und/oder in unterschiedlichen Höhen von der Oberfläche der Außenseite 12 abstehend ausgebildet sein.

Der Vorteil der Ausgestaltung des Grundkörpers 10 mit einem flexiblen, insbesondere flächigen Grundkörper ist, dass dieser sehr leicht in einer entsprechenden Fertigungsanlage individualisiert hergestellt werden kann. Dazu wird der Grundkörper 10 auf einen Trägerrahmen gespannt oder daran fixiert und in einen sogenannten 3D-Drucker eingeführt. Der 3D-Drucker arbeitet nach dem Prinzip des Polyjet-3D-Druckes, bei dem ein aushärtbares Polymer über einen Druckkopf mit mehreren Düsen auf das Werkstück, in dem Ausführungsbeispiel ein Textil, aufgetragen wird. Der Auftrag erfolgt in einem flüssigen Zustand durch die jeweiligen Düsen. Nach dem Auftragen aus den Düsen wird das noch flüssige Material der Strukturelemente ausgehärtet, insbesondere über UV-Licht, wobei sowohl die Viskosität des aufgetragenen Materials als auch die Einwirkdauer und der Einwirkzeitpunkt des UV-lichtes so abgestimmt wird, dass die erste Schicht, die unmittelbar in Kontakt mit dem Material des Grundkörpers 10 tritt, zwar in das Material eindringt, dieses jedoch nicht durchdringt. Bei einem stationären Druckkopf fährt dann die Trägervorrichtung für den Grundkörper um die Dicke einer aufgetragenen oder aufzutragenden Schicht nach unten. Eine Schichtdicke beträgt beispielsweise zwischen 10µm und 40 µm. Nach einer ausreichenden Zeitdauer der Aushärtung wird eine weitere Schicht aufgetragen, die dann wieder ausgehärtet wird. Werden mehrere Druckköpfe oder Düsen eingesetzt, können verschiedene Materialien und verschiedene Farben gedruckt werden. Die Druckköpfe und Düsen können auch in einer Ebene parallel zu der Aufspannfläche des Grundkörpers 10 relativ verfahrbar sein, sei es durch einen verfahrbaren Druckkopf oder durch einen verfahrbaren Träger. Dadurch ist es möglich, nahezu beliebige Formen der Strukturelemente zu erreichen. Über ein Computerprogramm gesteuert wird vorteilhafterweise Photopolymerharz aufgebracht, das in entsprechenden Kartuschen in der Druckvorrichtung vorhanden ist. Es kann aus mehreren Düsen gleichzeitig oder auch nacheinander gedruckt werden. Jede Düse kann Material mit einer eigenen, von den anderen Farben abweichenden Farbe, abweichenden Härte und anderen abweichenden Eigenschaften ausgestattet sein, sodass gleichzeitig unterschiedliche Farben und Materialeigenschaften aufgebracht und ausgehärtet werden können. In einer Ausgestaltung des aufgedruckten Materials können alle Farben erzeugt werden, beispielsweise über ein CMYK-System an Farbkomponenten, die gegebenenfalls gemischt werden.

Für jeden Grundkörper können unterschiedliche Muster, Farben und Materialien individuell mittels eines Computerprogramms erzeugt und auf den Grundkörper aufgebracht werden. Durch die Bedruckung in der Ebene wird ein einfacher Prozess gewährleistet. Der Grundkörper kann bereits in der endgültigen Form, mit der er an die Prothesenrichtung angebracht werden soll, gedruckt werden. Alternativ wird aus einem vorzugsweise rechteckigen Grundmaterial die benötigte Form des Grundkörpers ausgeschnitten. Gegebenenfalls sind bereits Befestigungseinrichtungen an dem Grundkörper vor dem Bedrucken vorhanden, beispielsweise Klettverschlüsse oder Magneteinrichtungen, sodass der Grundkörper nur noch um die Prothesenkomponente herumgelegt werden muss. Alternativ kann der Grundkörper ebenfalls in der Ebene bedruckt und dann auf einen Träger, beispielsweise einen insbesondere schalenförmigen Träger oder einen Protektor aufgebracht werden, beispielsweise aufgeklebt, aufgeklemmt oder über Klettverschlüsse reversibel daran festgelegt werden. Über den Träger kann auch die Befestigung an der Prothesenkomponente erfolgen, beispielsweise über Einklemmen, Einrasten, über eine Clipsverbindung, über eine Schraubverbindung oder dergleichen. Der Träger ist dann auch eine Befestigungseinrichtung oder Teil einer Befestigungseinrichtung.

Der Grundkörper 10 mit den darauf aufgebrachten Strukturelementen kann auf bereits bestehende Komponenten einer Protheseneinrichtung aufgeklebt oder anderweitig daran festgelegt werden. Die Klebeschicht ist dann die Befestigungseinrichtung.

## Patentansprüche

1. Prothesenabdeckung mit zumindest einer Befestigungseinrichtung (20) zum Festlegen der Prothesenabdeckung (1) an einer Prothesenkomponente (2) und einem flexiblen Grundkörper (10), mit einer der Prothesenkomponente (2) im angelegten Zustand zugewandten Innenseite (11) und einer der Innenseite (11) gegenüberliegenden Außenseite (12), an dem die zumindest eine Befestigungseinrichtung (20) angeordnet ist, **dadurch gekennzeichnet, dass** auf der Außenseite (12) zumindest ein von der Außenseite (12) abstehendes Strukturelement (30) in einem additiven Fertigungsverfahren aufgebracht ist.

2. Prothesenabdeckung nach Anspruch 1, **dadurch gekennzeichnet, dass** mehrere Strukturelemente (30) voneinander beabstandet an dem Grundkörper (10) aufgebracht sind.

3. Prothesenabdeckung nach Anspruch 1 oder 2, **dadurch gekennzeichnet, dass** die Strukturelemente (30) oder Gruppen von Strukturelementen (30) in unterschiedlichen Abständen zueinander und/oder mit unterschiedlichen Höhen auf dem Grundkörper (10) angeordnet sind.

4. Prothesenabdeckung nach einem der voranstehenden Ansprüche, **dadurch gekennzeichnet, dass** die Strukturelemente (30) in regelmäßigen und/oder unregelmäßigen Formen, insbesondere als Halbkugeln, Kegel, Pyramiden, Kegelstümpfe, Pyramidenstümpfe, in Tropfenform, als Platten, Würfel und/oder Noppen ausgebildet sind.

5. Prothesenabdeckung nach einem der voranstehenden Ansprüche, **dadurch gekennzeichnet, dass** die Strukturelemente (30) mehrfarbig und/oder aus unterschiedlichen Materialien ausgebildet sind.

6. Prothesenabdeckung nach einem der voranstehenden Ansprüche, **dadurch gekennzeichnet, dass** die Strukturelemente (30) aus einem elastischen Material ausgebildet sind.

7. Prothesenabdeckung nach einem der voranstehenden Ansprüche, **dadurch gekennzeichnet, dass** die Strukturelemente (30) eine durchsichtige oder teildurchsichtige oder vollfarbige Außenschicht aufweisen.

8. Prothesenabdeckung nach einem der voranstehenden Ansprüche, **dadurch gekennzeichnet, dass** das Material des Strukturelementes (30) den Grundkörper (10) nicht durchdringt.

9. Prothesenabdeckung nach einem der voranstehenden Ansprüche, **dadurch gekennzeichnet, dass** der Grundkörper (10) elastisch und/oder aufnahmefähig für das mit dem Grundkörper (10) in Kontakt stehende Material der Strukturelemente (30) ist.

10. Prothesenabdeckung nach einem der voranstehenden Ansprüche, **dadurch gekennzeichnet, dass** der Grundkörper (10) mehrere Abschnitte (15) aus unterschiedlichen Materialen aufweist.

11. Prothesenabdeckung nach Anspruch 10, **dadurch gekennzeichnet, dass** Abschnitte (15) aus unterschiedlichen Materialien unterschiedliche Anordnungen und/oder Formen von Strukturelementen (30) aufweisen.

12. Prothesenabdeckung nach einem der voranstehenden Ansprüche, **dadurch gekennzeichnet, dass** der Grundkörper (10) einen proximalen und/oder distalen Halter (40) oder Rahmen aufweist und/oder auf oder an einem Träger (45) angeordnet ist.

13. Prothesenabdeckung nach Anspruch 12, **dadurch gekennzeichnet, dass** der Grundkörper (10) auf dem Träger (45) aufgeklebt oder lösbar daran über die Befestigungseinrichtung (20) festgelegt ist.

14. Prothesenabdeckung nach Anspruch 12, **dadurch gekennzeichnet, dass** der Halter (40) an dem Grundkörper (10) angenäht, angeklebt, angeschweißt, angespritzt oder umspritzt ist.

15. Prothesenabdeckung nach einem der voranstehenden Ansprüche, **dadurch gekennzeichnet, dass** die Befestigungseinrichtung (20) als Formschlusselement und/oder Kraftschlusselement ausgebildet ist.

16. Verfahren zum Herstellen einer Prothesenabdeckung mit zumindest einer Befestigungseinrichtung (20) zum Festlegen der Prothesenabdeckung an einer Prothesenkomponente und einem flexiblen Grundkörper, mit einer der Prothesenkomponente im angelegten Zustand zugewandten Innenseite und einer der Innenseite gegenüberliegenden Außenseite (12), an dem die zumindest eine Befestigungseinrichtung (20) angeordnet ist, **dadurch gekennzeichnet, dass** auf der Außenseite (12) zumindest ein von der Außenseite (12) abstehendes Strukturelement (30) in einem additiven Fertigungsverfahren aufgebracht wird.

17. Verfahren nach Anspruch 16, **dadurch gekennzeichnet, dass** der Grundkörper (10) mit dem zumindest einem Strukturelement (30) an einem Halter (40) oder Träger (45) befestigt wird.

## Claims

1. A prosthesis cover comprising at least one fastening device (20) for fixing the prosthesis cover (1) to a prosthesis component (2) and a flexible main body (10) having an inner side (11), which faces the prosthesis component (2) in the placed-on state, and an outer side (12), which is situated opposite the inner side (11) and on which the at least one fastening device (20) is arranged, **characterized in that** at least one structural element (30) is applied to the outer side (12) in an additive manufacturing process and protrudes from the outer side (12).

2. The prosthesis cover as claimed in claim 1, **characterized in that** multiple structural elements (30) are applied at a spacing from one another on the main body (10).

3. The prosthesis cover as claimed in claim 1 or 2, **characterized in that** the structural elements (30) or groups of structural elements (30) are arranged at different spacings from one another and/or with different heights on the main body (10).

4. The prosthesis cover as claimed in one of the preceding claims, **characterized in that** the structural elements (30) are formed in regular and/or irregular shapes, in particular as hemispheres, cones, pyramids, truncated cones, truncated pyramids, in droplet form, as plates, cubes and/or studs.

5. The prosthesis cover as claimed in one of the preceding claims, **characterized in that** the structural elements (30) are multicolored and/or made of different materials.

6. The prosthesis cover as claimed in one of the preceding claims, **characterized in that** the structural elements (30) are made of an elastic material.

7. The prosthesis cover as claimed in one of the preceding claims, **characterized in that** the structural elements (30) have a transparent or semitransparent or fullcolor outer layer.

8. The prosthesis cover as claimed in one of the preceding claims, **characterized in that** the material of the structural element (30) does not penetrate the main body (10).

9. The prosthesis cover as claimed in one of the preceding claims, **characterized in that** the main body (10) is elastic and/or retentive for the material of the structural elements (30) that is in contact with the main body (10).

10. The prosthesis cover as claimed in one of the preceding claims, **characterized in that** the main body (10) has multiple portions (15) made of different materials.

11. The prosthesis cover as claimed in claim 10, **characterized in that** portions (15) made of different materials have different arrangements and/or shapes of structural elements (30).

12. The prosthesis cover as claimed in one of the preceding claims, **characterized in that** the main body (10) has a proximal and/or distal holder (40) or frame and/or is arranged on a support (45).

13. The prosthesis cover as claimed in claim 12, **characterized in that** the main body (10) is adhesively bonded on the support (45), or fixed detachably thereto via the fastening device (20).

14. The prosthesis cover as claimed in claim 12, **characterized in that** the holder (40) is sewn, adhesively bonded, welded, integrally molded or overmolded on the main body (10).

15. The prosthesis cover as claimed in one of the preceding claims, **characterized in that** the fastening device (20) is in the form of a form-fitting element and/or forcefitting element.

16. A process for producing a prosthesis cover comprising at least one fastening device (20) for fixing the prosthesis cover to a prosthesis component and a flexible main body having an inner side, which faces the prosthesis component in the placed-on state, and an outer side (12), which is situated opposite the inner side and on which the at least one fastening device (20) is arranged, **characterized in that** at least one structural element (30) is applied to the outer side (12) in an additive manufacturing process and protrudes from the outer side (12).

17. The process as claimed in claim 16, **characterized in that** the main body (10) with the at least one structural element (30) is fastened to a holder (40) or support (45).

## Revendications

1. Élément de couverture de prothèse comportant au moins un dispositif de fixation (20) destiné à fixer l'élément de couverture de prothèse (1) à un composant prothétique (2) et un corps de base flexible (10), présentant une face intérieure (11) tournée vers le composant prothétique (2) à l'état mis en place (11) et une face extérieure (12) opposée à la face intérieure (11), sur laquelle est agencé ledit au moins un dispositif de fixation (20),
**caractérisé en ce qu'**au moins un élément structurel (30) faisant saillie de la face extérieure (12) est appliqué sur la face extérieure (12) par un procédé de fabrication additive.

2. Élément de couverture de prothèse selon la revendication 1,
**caractérisé en ce que** plusieurs éléments structurels (30) sont appliqués sur le corps de base (10) à distance les uns des autres.

3. Élément de couverture de prothèse selon la revendication 1 ou 2,
**caractérisé en ce que** les éléments structurels (30) ou des groupes d'éléments structurels (30) sont agencés sur le corps de base (10) à des distances différentes les uns des autres et/ou à des hauteurs différentes.

4. Élément de couverture de prothèse selon l'une des revendications précédentes,
**caractérisé en ce que** les éléments structurels (30) se présentent sous des formes régulières et/ou irrégulières, en particulier sous forme de demisphères, de cônes, de pyramides, de troncs de cône, de troncs de pyramide, de gouttes, de plaques, de cubes et/ou de picots.

5. Élément de couverture de prothèse selon l'une des revendications précédentes,
**caractérisé en ce que** les éléments structurels (30) sont multicolores et/ou constitués de différents matériaux.

6. Élément de couverture de prothèse selon l'une des revendications précédentes,
**caractérisé en ce que** les éléments structurels (30) sont réalisés dans un matériau élastique.

7. Élément de couverture de prothèse selon l'une des revendications précédentes,
**caractérisé en ce que** les éléments structurels (30) présentent une couche extérieure transparente ou partiellement transparente ou en couleur.

8. Élément de couverture de prothèse selon l'une des revendications précédentes,
**caractérisé en ce que** le matériau de l'élément structurel (30) ne traverse pas le corps de base (10).

9. Élément de couverture de prothèse selon l'une des revendications précédentes,
**caractérisé en ce que** le corps de base (10) est élastique et/ou capable d'absorber le matériau des éléments structurels (30) en contact avec le corps de base (10).

10. Élément de couverture de prothèse selon l'une des revendications précédentes,
**caractérisé en ce que** le corps de base (10) comporte plusieurs sections (15) constituées de matériaux différents.

11. Élément de couverture de prothèse selon la revendication 10,
**caractérisé en ce que** les sections (15) constituées de matériaux différents présentent des agencements et/ou des formes différents d'éléments structurels (30).

12. Élément de couverture de prothèse selon l'une des revendications précédentes,
**caractérisé en ce que** le corps de base (10) comporte un support (40) ou un cadre proximal et/ou distal et/ou est agencé sur un élément porteur (45) ou au niveau de celui-ci.

13. Élément de couverture de prothèse selon la revendication 12,
**caractérisé en ce que** le corps de base (10) est collé sur l'élément porteur (45) ou y est fixé de manière amovible à l'aide du dispositif de fixation (20).

14. Élément de couverture de prothèse selon la revendication 12,
**caractérisé en ce que** le support (40) est cousu, collé, soudé, moulé par injection ou surmoulé sur le corps de base (10).

15. Élément de couverture de prothèse selon l'une des revendications précédentes,
**caractérisé en ce que** le dispositif de fixation (20) est conçu comme un élément de liaison par complémentarité de forme et/ou un élément de liaison par adhérence.

16. Procédé de fabrication d'un élément de couverture de prothèse comportant au moins un dispositif de fixation (20) destiné à fixer l'élément de couverture de prothèse à un composant prothétique et un corps de base flexible, présentant une face intérieure tournée vers le composant prothétique à l'état mis en place et une face extérieure (12) opposée à la face intérieure, sur laquelle est agencé ledit au moins un dispositif de fixation (20), **caractérisé en ce qu'**au moins un élément structurel (30) faisant saillie de la face extérieure (12) est appliqué sur la face extérieure (12) par un procédé de fabrication additive.

17. Procédé selon la revendication 16,
**caractérisé en ce que** le corps de base (10), muni dudit au moins un élément structurel (30), est fixé à un support (40) ou à un élément porteur (45).
